# EUROPEAN PATENT APPLICATION

(11) **EP 4 520 822 A1**
(43) Date of publication of application: **12.03.2025**
(21) Application number: 23799487.6
(22) Date of filing: 01.05.2023
(51) Int. Cl.: C12N 15/11, C12Q 1/6813, C12Q 1/686, C12Q 1/6883

(54) **DISEASE STATE ASSESSMENT KIT FOR RETT SYNDROME AND USE OF GENE CONTAINED IN MITOCHONDRIAL GENOME AS BIOMARKER**

(30) Priority: 06.05.2022 JP 2022076396
(71) Applicant: Institute of Rheological Function of Food Co., Ltd., Kasuya-gun, Fukuoka 811-2501 (JP); Kyushu University, National University Corporation, Nishi-ku Fukuoka-shi Fukuoka 819-0395 (JP)
(72) Inventor: FUJINO Takehiko, Fukuoka-shi, Fukuoka 813-0043 (JP); MAWATARI Shiro, Fukuoka-shi, Fukuoka 813-0016 (JP); HONSHO Masanori, Fukuoka-shi, Fukuoka 819-0395 (JP)
(74) Representative: V.O.
(86) International application number: PCT/JP2023/017009
(87) International publication number: WO 2023/214557

(57) **Abstract**

A kit for determining a disease state of Rett syndrome includes a reagent for detecting a gene included in the mitochondrial genome, in a sample collected from a subject.

## Description

### Technical Field

The present disclosure relates to a kit for determining a disease state of Rett syndrome, and use of a gene included in the mitochondrial genome as a biomarker.

### Background Art

Rett syndrome (hereinafter also referred to as "RTT") is a progressive neurodevelopmental disorder caused by mutation of the MeCP2 gene that is located on the X chromosome. Development of RTT occurs mainly in female infants. The patients show normal growth for about six months to one year after birth, and thereafter, they show various neurological symptoms such as autistic tendencies.

Toll-like receptor (TLR) 9 is known to be involved in inflammation in the brain of RTT model mice lacking the MeCP2 gene (see Non Patent Literature 1). TLR induces Th1 mainly in response to microbial infection, and is involved in innate immunity together with NK cells and others. TLR9 is known to recognize unmethylated CpG motifs in viral DNAs and bacterial DNAs. Non Patent Literature 2 reports that mutation of the MeCP2 gene causes a decrease in the mitochondrial function, and that the mitochondrial function is impaired in RTT.

### Citation List

### Non Patent Literature

Non Patent Literature 1: Hideyuki Nakashima, "Research Grant Report (2019): Elucidation of the mechanism of development of Rett syndrome due to microglial activation via an endogenous ligand, and establishment of a therapeutic method for Rett syndrome", [online], June 1, 2020, Rett Syndrome Organization Japan, [retrieved on April 22, 2022], Internet <URL: https://www.npo-rett.jp/rett_img/rett_zyosei_p/nakashima_hokoku.pdf>
Non Patent Literature 2: Natalya Shulyakova and three others, "Mitochondrial Dysfunction in the Pathogenesis of Rett Syndrome: Implications for Mitochondria-Targeted Therapies", Frontiers in cellular neuroscience, 2017, 11, 58
Non Patent Literature 3: Shin Nabatame and another, "Rett Syndrome: Revised Diagnostic Criteria (2010 edition)", Guidebook for Diagnosis of Rett syndrome, Osaka University Press, March 23, 2015, pp. 21-23

### Summary of Invention

### Technical Problem

Diagnosis of RTT requires the presence of an episode of regression, and satisfaction of all major diagnostic criteria and exclusion diagnostic criteria. The major diagnostic criteria are loss of function of hands, spoken language communication, abnormality of gait, and the presence or absence of a stereotyped hand behavior (see Non Patent Literature 3). Thus, diagnosis of RTT is based primarily on behavioral observations. For simple evaluation of the degree of progression of the disease state of RTT, and evaluation of recovery from the disease state of RTT, biomarkers associated with the disease state of RTT have been demanded.

The present disclosure was carried out under such circumstances, and an objective of the present disclosure is to provide a kit for determining a disease state of Rett syndrome capable of simple determination of the disease state of RTT, and use of a gene included in the mitochondrial genome as a biomarker.

### Solution to Problem

Focusing on the fact that the CpG motifs in the mitochondrial genome are hardly modified by methylation, the present inventors intensively studied based on the hypothesis that a gene included in the mitochondrial genome, or a fragment thereof, functions as a ligand of TLR9. As a result, the present inventors discovered that, in cases where MeCP2 expression is suppressed or knocked out, genes included in the mitochondrial genome are detected in larger amounts in blood and the like, thereby completing the present disclosure.

A kit for determining a disease state of Rett syndrome according to a first aspect of the present disclosure includes:
a reagent for detecting a gene included in the mitochondrial genome, in a sample collected from a subject.

The reagent may contain:
a primer for amplifying at least part of a base sequence of the gene in polymerase chain reaction in which the base sequence serves as a template; and a probe that hybridizes with at least part of a base sequence of an amplification product amplified by the polymerase chain reaction.

The kit for determining a disease state of Rett syndrome according to the first aspect of the present disclosure may further include:
a labeling substance that indicates a fact that the probe has hybridized with the base sequence of the amplification product.

The gene may be selected from the group consisting of a gene encoding 16S ribosomal RNA, a gene encoding cytochrome c oxidase subunit 1, a gene encoding cytochrome c oxidase subunit 2, a gene encoding cytochrome c oxidase subunit 3, a gene encoding cytochrome b, a gene encoding NADH dehydrogenase 1, a gene encoding NADH dehydrogenase 2, a gene encoding NADH dehydrogenase 3, a gene encoding NADH dehydrogenase 4, a gene encoding NADH dehydrogenase 4L, a gene encoding NADH dehydrogenase 5, a gene encoding NADH dehydrogenase 6, a gene encoding ATP synthase 6, and a gene encoding ATP synthase 8.

Use according to a second aspect of the present disclosure is
use of a gene included in mitochondrial genome as a biomarker to assist determination of a disease state of Rett syndrome in a subject.

### Advantageous Effects of Invention

By the present disclosure, a disease state of Rett syndrome can be simply determined.

### Brief Description of Drawings

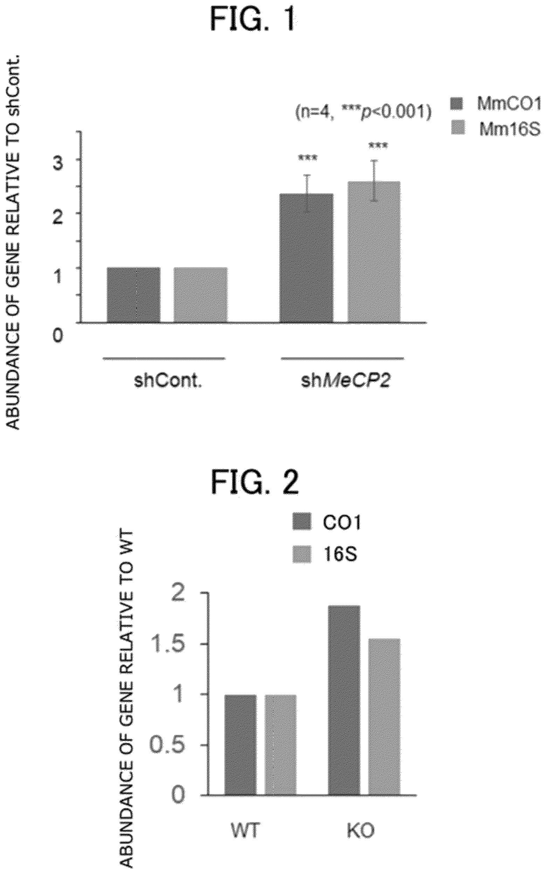

FIG. 1 is a diagram illustrating the relative abundances of the gene encoding 16S ribosomal RNA (*16S*) and the gene encoding cytochrome c oxidase subunit 1 *(CO1)* in a medium obtained by culturing cells in which expression of MeCP2 was suppressed by shRNA;
FIG. 2 is a diagram illustrating the relative abundances of *16S* and *CO1* in serum of an MeCP2 knockout mouse that is an RTT model;

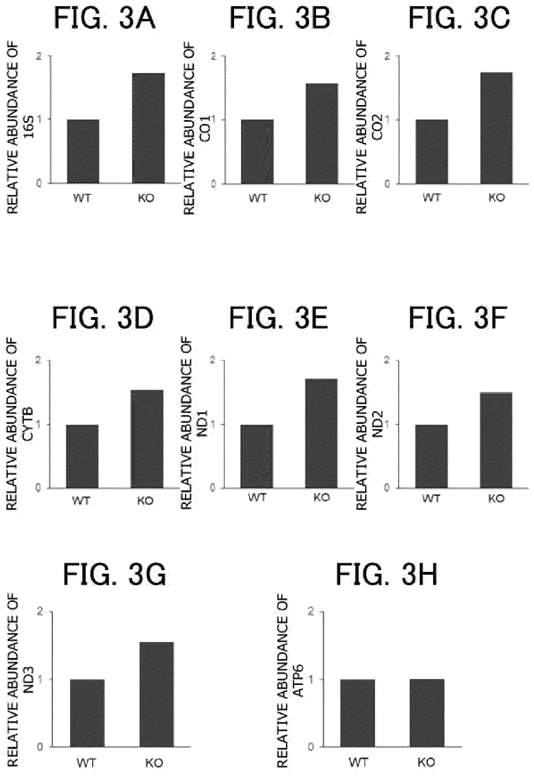

FIG. 3 is a diagram illustrating the relative abundances of genes included in the mitochondrial genome in plasma of an MeCP2 knockout mouse. FIG. 3A, FIG. 3B, FIG. 3C, FIG. 3D, FIG. 3E, FIG. 3F, FIG. 3G and FIG. 3H are diagrams illustrating the relative abundances of *16S, CO1,* the gene encoding cytochrome c oxidase subunit 2 *(CO2),* the gene encoding cytochrome b (*CYTB*)*,* the gene encoding NADH dehydrogenase 1 (*ND1*)*,* the gene encoding NADH dehydrogenase 2 (*ND2*)*,* the gene encoding NADH dehydrogenase 3 (*ND3*), and the gene encoding ATP synthase 6 *(ATP6);* and

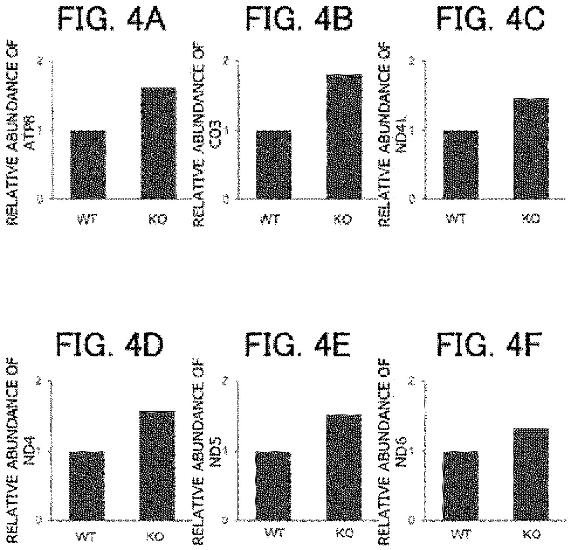

FIG. 4 is a diagram illustrating the relative abundances of genes included in the mitochondrial genome in plasma of an MeCP2 knockout mouse. FIG. 4A, FIG. 4B, FIG. 4C, FIG. 4D, FIG. 4E, and FIG. 4F are diagrams illustrating the relative abundances of the gene encoding ATP synthase 8 (*ATP8*), the gene encoding cytochrome c oxidase subunit 3 (*CO3*), the gene encoding NADH dehydrogenase 4L (*ND4L*), the gene encoding NADH dehydrogenase 4 (*ND4*), the gene encoding NADH dehydrogenase 5 (*ND5*), and the gene encoding NADH dehydrogenase 6 (*ND6*)*.*

### Description of Embodiments

Embodiments according to the present disclosure are described below with reference to drawings. The present disclosure is not limited by the following embodiments and drawings. In the following embodiments, expressions such as "have", "include", or "contain" also encompass the meaning of "consist of" or "be constituted by".

### Embodiment 1

The kit for determining a disease state of RTT according to the present embodiment includes a reagent for detecting a gene included in the mitochondrial genome. Mitochondria are organelles of eukaryotic cells. Mitochondria are organelles that generate energy through oxidative phosphorylation. There is a plurality of mitochondria in a cell, and the mitochondria themselves have a plurality of mitochondrial genomes (also called "mitochondrial DNA"). Thus, a cell has a plurality of mitochondrial genomes that is not necessarily identical to each other.

Since mutation of the MeCP2 gene causes a decrease in the mitochondrial function, and since the mitochondrial function is impaired in RTT, DNA containing an unmethylated CpG motif derived from the mitochondrial genome is thought to bind to TLR9, to induce inflammation in the brain. Therefore, genes included in the mitochondrial genome can be indices of a disease state of RTT.

The gene to be detected is not limited as long as it is included in the mitochondrial genome. Examples of the gene include the gene encoding 12S ribosomal RNA, *16S, CO1, CO2, CO3, CYTB, ND1, ND2, ND3, ND4, ND4L, ND5, ND6, ATP6, ATP8,* and the genes encoding the 22 kinds of tRNAs.

The reagent according to the present embodiment detects the above gene in a sample collected from a subject. The subject is, more specifically, a human. The subject is preferably a human suspected of having RTT, or an RTT patient. The sample may be any biological sample, and examples of the sample include body fluids such as blood, serum, bone marrow fluid, saliva, semen, peritoneal fluid, and urine; and cells such as those of liver and skin; of the subject. DNA contained in the sample can be extracted and purified by known methods.

Preferably, the reagent contains: a primer for amplifying at least part of a base sequence of the gene in polymerase chain reaction (PCR) in which the base sequence serves as a template; and a probe that hybridizes with at least part of a base sequence of an amplification product amplified by the PCR. The at least part of a base sequence of the gene is preferably a unique base sequence that is included in the gene, but not included in other genes. The primer used in the PCR is designed, for example, to amplify at least one of nucleic acids having a base sequence of at least not less than 10 consecutive bases, preferably nucleic acids having a base sequence of 50 to 200 consecutive bases, more preferably nucleic acids having a base sequence of 100 to 150 consecutive bases, and nucleic acids complementary thereto, in the gene.

The amplification product is detected or quantified utilizing a probe that hybridizes with at least part of the base sequence of the amplification product. A probe means a nucleic acid fragment for analysis of a specific site in a nucleic acid or a base sequence thereof by utilization of hybridization or the like based on the complementarity of the nucleic acid. The probe is, for example, an oligonucleotide that hybridizes with a base sequence of at least not less than 10 consecutive bases, preferably a base sequence of 10 to 100 consecutive bases, more preferably a base sequence of 10 to 50 consecutive bases, or a base sequence complementary thereto, in the amplification product.

As long as the probe hybridizes with the amplification product, and does not hybridize with nucleic acids other than the amplification product, the base sequence of the probe does not need to be completely complementary to the base sequence of the amplification product, and may be a base sequence having one or more of substitutions, deletions, and additions compared to the sequence that is completely complementary to the base sequence of the amplification product.

In order to quantitatively detect the above gene included in the mitochondrial genome, the kit for determining a disease state of RTT according to the present embodiment preferably further includes a labeling substance that indicates the fact that the probe has hybridized with the base sequence of the amplification product. Examples of the labeling substance include fluorescent dyes and radioactive substances. The labeling substance is preferably a fluorescent dye for use in the quantification of an amplification product amplified by PCR. The amplification product can be quantified, through the fluorescent dye, by a known method such as the intercalation method, the hybridization method, or the LUX method. The intercalation method uses a fluorescent dye that emits fluorescence upon its specific insertion into double-stranded DNA. The hybridization method uses a probe modified with a fluorescent dye.

The hybridization is carried out under stringent conditions that allow the probe to hybridize with the amplification product, but does not allow the probe to hybridize with nucleic acids other than the amplification product. The stringent conditions can be appropriately determined according to, for example, Molecular Cloning: A Laboratory Manual, 3rd Edition (2001). Under the stringent conditions, incubation is performed, for example, in 0.2 × SSC and 0.1% SDS at 65°C.

A method of using the kit for determining a disease state of RTT according to the present embodiment is described below. First, a gene included in the mitochondrial genome in a sample collected from a subject is detected using the above reagent.

In cases where the hybridization of the probe with the amplification product is detected through a labeling substance, the amount of the amplification product generated can be determined by measuring a signal of the labeling substance. The amount of the amplification product generated correlates with the abundance of the above gene included in the mitochondrial genome, originally contained in the sample. Thus, the disease state of RTT in the subject can be determined based on the amount of the amplification product generated. In the determination of the disease state, for example, the measured value is compared with a predetermined reference value. As the reference value, for example, the average of measured values of non-RTT subjects may be used. After defining degrees of the progression of the disease state of RTT, the average of measured values of subjects corresponding to each degree of progression may be determined in advance. By comparing the reference value determined for each degree of progression with the measured value of the subject for whom the disease state is to be determined, the degree of progression of the disease state of this subject can be determined.

By the kit for determining a disease state of RTT according to the present embodiment, the disease state of RTT in a subject can be simply determined by detecting a gene included in the mitochondrial genome in a sample collected from the subject.

As described above, the presence or absence, or the abundance, of a gene included in the mitochondrial genome, in a sample collected from the subject is useful information for determining a disease state of Rett syndrome. Thus, another embodiment provides use of a gene included in the mitochondrial genome as a biomarker to assist determination of a disease state of Rett syndrome in a subject. Another embodiment provides a method of determining a disease state of RTT. The method of determining a disease state of RTT includes: a quantification step of quantifying a gene included in the mitochondrial genome in a sample collected from a subject using the reagent; and a determination step of determining a disease state of RTT in the subject based on the amount of the gene. Also provided is a therapeutic method for RTT, the method including: a quantification step; a determination step; and an administration step of administering a therapeutic agent for RTT to the subject depending on the disease state of RTT determined in the determination step.

### Examples

The present disclosure is described more concretely by way of the following Examples. However, the present disclosure is not limited by Examples.

### Test Example 1

### [Establishment of shRNA-Expressing Cell Line]

In the following, a vector that expresses shRNA targeting mouse MeCP2 (NM_010788) (catalog number: TRCN0000304464, Sigma) was used as "shMeCP2". An amplification product from shMeCP2 was obtained by PCR to prepare a vector excluding the MeCP2-targeting sequence. The vector was designated as "shCont.". The PCR was carried out using a forward primer and a reverse primer for shMeCP2 having the base sequences of SEQ ID NO:1 and SEQ ID NO:2, respectively.

shMeCP2 or shCont. was introduced into BV2 cells (derived from mouse glial cells) cultured in DMEM (manufactured by Sigma) supplemented with 10% fetal calf serum (FCS, manufactured by Sigma). By utilization of the puromycin resistance gene included in the vector, puromycin-resistant lines (concentration of puromycin, 1 µg/ml) were separated. The resulting cells were designated BV2/shMeCP2 and BV2/shCont.

### [Recovery of Genome from Medium of shRNA-Expressing Cell Line, and Quantification of Genes Included in Mitochondrial Genome]

The media of BV2/shMeCP2 and BV2/shCont. were collected, and centrifuged at 1000 × g for 5 minutes. From the resulting supernatants, genome was recovered using a DNeasy Blood & Tissue Kit (manufactured by QIAGEN). Subsequently, real-time PCR was carried out using the ABI7500 Real Time System (manufactured by Applied Biosystem) with SYBR Premix Ex Taq (trademark) II (Ti RNaseH Plus) (manufactured by Takara Bio Inc.). In the real-time PCR, the hexokinase 2 gene (*HK2*), included in nuclear DNA, and *16S* and *CO1,* included in the mitochondrial genome, were quantified. The real-time PCR for *HK2* was carried out using a forward primer and a reverse primer having the base sequences of SEQ ID NO:3 and SEQ ID NO:4, respectively. The real-time PCR for *16S* was carried out using a forward primer and a reverse primer having the base sequences of SEQ ID NO:5 and SEQ ID NO:6, respectively. The real-time PCR for *CO1* was carried out using a forward primer and a reverse primer having the base sequences of SEQ ID NO:7 and SEQ ID NO:8, respectively.

### (Results)

FIG. 1 illustrates the relative abundances of *16S* and *CO1* standardized by the abundance of *HK2,* in the media of BV2/shMeCP2 and BV2/shCont. Due to suppression of the expression of MeCP2, the abundances of *16S* and *CO1* increased.

### Test Example 2

### [Recovery of Genome from Serum of MeCP2-Knockout (MeCP2-KO) Mouse, and Quantification of Genes Included in Mitochondrial Genome]

A male 8-week-old wild-type mouse (WT), and a 7-week-old MeCP2-KO mouse 003890 (B6.129P2(C)-Mecp2tm1.1Bird/J, The Jackson Laboratory) that is an RTT model mouse, were subjected to cardiac blood collection under anesthesia with pentobarbital (injection: 0.1 to 0.2 ml). The blood was left to stand on ice for 30 minutes, and then centrifuged at 4°C for 10 minutes at 3000 rpm. The serum fraction of the resulting supernatant was collected. Subsequently, genome was recovered from 50 µl of the serum using a DNeasy Blood & Tissue Kit (manufactured by QIAGEN). The recovered genome was subjected to real-time PCR in the same manner as in Test Example 1 above, to quantify *16S* and *CO1* in the serum of each mouse.

### (Results)

FIG. 2 illustrates the relative abundances of *16S* and *CO1* standardized by the abundance of HK2, in the sera of WT and MeCP2-KO. MeCP2-KO showed higher abundances of *16S* and *CO1.*

### Test Example 3

[Recovery of Genome from Plasma of MeCP2-KO Mouse, and Quantification of Mitochondrial Genome]

A male 4-week-old WT and an MeCP2-KO mouse 003890 were subjected to cardiac blood collection using a 1-ml syringe filled with 5000 units/5 ml heparin Na, under anesthesia with pentobarbital (injection: 0.1 to 0.2 ml). The blood was left to stand on ice for 30 minutes, and then subjected to centrifugation at 4°C for 30 minutes at 3000 rpm to collect the plasma fraction of the resulting supernatant. Subsequently, genome was recovered from 50 µl of the serum using a DNeasy Blood & Tissue Kit (manufactured by QIAGEN). The recovered genome was subjected to real-time PCR in the same manner as in Test Example 1 above, to quantify *CO2, CO3, CYTB, ND1, ND2, ND3, ND4, ND4L, ND5, ND6, ATP6, and ATP8* as well as *16S* and *CO1* in the plasma of each mouse. In Test Example 3, the abundance of each gene included in the mitochondrial genome was not standardized by the abundance of *HK2.*

A forward primer and a reverse primer having the base sequences of SEQ ID NO:9 and SEQ ID NO:10, respectively, were used for *CO2.* A forward primer and a reverse primer having the base sequences of SEQ ID NO: 11 and SEQ ID NO:12, respectively, were used for *CO3.* A forward primer and a reverse primer having the base sequences of SEQ ID NO:13 and SEQ ID NO:14, respectively, were used for *CYTB.* A forward primer and a reverse primer having the base sequences of SEQ ID NO:15 and SEQ ID NO:16, respectively, were used for *ND1.* A forward primer and a reverse primer having the base sequences of SEQ ID NO:17 and SEQ ID NO:18, respectively, were used for *ND2.* A forward primer and a reverse primer having the base sequences of SEQ ID NO:19 and SEQ ID NO:20, respectively, were used for *ND3.* A forward primer and a reverse primer having the base sequences of SEQ ID NO:21 and SEQ ID NO:22, respectively, were used for *ND4.* A forward primer and a reverse primer having the base sequences of SEQ ID NO:23 and SEQ ID NO:24, respectively, were used for *ND4L.* A forward primer and a reverse primer having the base sequences of SEQ ID NO:25 and SEQ ID NO:26, respectively, were used for *ND5.* A forward primer and a reverse primer having the base sequences of SEQ ID NO:27 and SEQ ID NO:28, respectively, were used for *ND6.* A forward primer and a reverse primer having the base sequences of SEQ ID NO:29 and SEQ ID NO:30, respectively, were used for *ATP6.* A forward primer and a reverse primer having the base sequences of SEQ ID NO:31 and SEQ ID NO:32, respectively, were used for *ATP8.*

### (Results)

FIG. 3A, FIG. 3B, FIG. 3C, FIG. 3D, FIG. 3E, FIG. 3F, FIG. 3G, and FIG. 3H illustrate the relative abundances of *16S, CO1, CO2, CYTB, ND1, ND2, ND3,* and *ATP6,* respectively, in MeCP2-KO relative to those in WT. FIG. 4A, FIG. 4B, FIG. 4C, FIG. 4D, FIG. 4E, and FIG. 4F illustrate the relative abundances of *ATP8, CO3, ND4L, ND4, ND5, and ND6,* respectively, in MeCP2-KO relative to those in WT. The abundances of these genes included in the mitochondrial genome were higher in MeCP2-KO relative to those in WT. Table 1 below shows details of the results of Test Example 3 that was carried out by n=3. In Table 1, "++" indicates an increase by 2-fold or more. "+" indicates an increase by 1.2-fold or more, but less than 2-fold. "-" indicates an increase by less than 1.2-fold.

**Table 1**

| | **16S** | **CO1** | **CO2** | **CYTB** | **ND1** | **ND2** | **ND3** | **ATP6** | **ATP8** | **CO3** | **ND4L** | **ND4** | **ND5** | **ND6** |
|---|---|---|---|---|---|---|---|---|---|---|---|---|---|---|
| Experiment 1 | ++ | ++ | ++ | ++ | ++ | ++ | ++ | ++ | ++ | ++ | ++ | ++ | ++ | ++ |
| Experiment 2 | + | + | + | + | + | + | + | + | + | + | + | + | + | + |
| Experiment 3 | + | + | + | + | + | + | - | + | + | + | + | + | + | + |

As shown in the above results, most of the genes included in the mitochondrial genome in plasma were more abundant in the MeCP2-KO mouse than in WT. Therefore, the other genes included in the mitochondrial genome are expected to exhibit similar dynamics.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2022-76396, filed on May 6, 2022, the entire disclosure of which is incorporated by reference herein.

### Industrial Applicability

The present disclosure is useful for determination of a disease state of Rett syndrome.

## Claims

1. A kit for determining a disease state of Rett syndrome, the kit comprising:
a reagent for detecting a gene included in mitochondrial genome, in a sample collected from a subject.

2. The kit for determining a disease state of Rett syndrome according to claim 1, wherein the reagent contains:
a primer for amplifying at least part of a base sequence of the gene in polymerase chain reaction in which the base sequence serves as a template; and
a probe that hybridizes with at least part of a base sequence of an amplification product amplified by the polymerase chain reaction.

3. The kit for determining a disease state of Rett syndrome according to claim 2, further comprising:
a labeling substance that indicates a fact that the probe has hybridized with the base sequence of the amplification product.

4. The kit for determining a disease state of Rett syndrome according to any one of claims 1 to 3, wherein
the gene is selected from the group consisting of a gene encoding 16S ribosomal RNA, a gene encoding cytochrome c oxidase subunit 1, a gene encoding cytochrome c oxidase subunit 2, a gene encoding cytochrome c oxidase subunit 3, a gene encoding cytochrome b, a gene encoding NADH dehydrogenase 1, a gene encoding NADH dehydrogenase 2, a gene encoding NADH dehydrogenase 3, a gene encoding NADH dehydrogenase 4, a gene encoding NADH dehydrogenase 4L, a gene encoding NADH dehydrogenase 5, a gene encoding NADH dehydrogenase 6, a gene encoding ATP synthase 6, and a gene encoding ATP synthase 8.

5. Use of a gene contained in mitochondrial genome as a biomarker to assist determination of a disease state of Rett syndrome in a subject.
